# EUROPEAN PATENT APPLICATION

(11) **EP 2 177 509 A2**
(43) Date of publication of application: **21.04.2010**
(21) Application number: 09172661.2
(22) Date of filing: 09.10.2009
(51) Int. Cl.: C07C 319/20, C07C 323/52

(54) **Process for producing 2-hydroxy-4-methylthiobutanoic acid**

(30) Priority: 10.10.2008 JP 2008263735
(71) Applicant: Sumitomo Chemical Company, Limited, Tokyo 104-8260 (JP)
(72) Inventor: Fujita, Kazuo, Ehime (JP); Onishi, Kozo, Ehime (JP)
(74) Representative: Winter, Brandl, Fürniss, Hübner Röss, Kaiser, Polte Partnerschaft Patent- und Rechtsanwaltskanzlei

(57) **Abstract**

The present invention discloses a use of a highly impact-resistant and corrosion-resistant material for an apparatus for use in hydrolysis of 2-hydroxy-4-methythiobutanamide to obtain 2-hydroxy-4-methythiobutanoic acid, said material being readily fabricated for an apparatus with a complicated structure and being an alloy which contains 16.0 to 22.0% by weight of a Cr element, 16.0 to 22.0% by weight of a Mo element, 1.0 to 2.5% by weight of a Ta element and a Ni element as the rest, an alloy which contains 26.0 to 32.0% by weight of a Mo element and a Ni element as the rest, or an alloy which contains 0.12 to 0.25% by weight of a Pd element and a Ti element as the rest.

## Description

### BACKGROUND OF THE INVENTION

### Field of the Invention

The present application is filed, claiming the Paris Convention priorities of Japanese Patent Application No. 2008-263735 (filed on October 10, 2008), the entire content of which is incorporated herein by reference.
The present invention relates to a process for producing 2-hydroxy-4-methylthiobutanoic acid from 2-hydroxy-4-methylthiobutanenitrile via 2-hydroxy-4-methylthiobutanamide. In particular, the invention relates to a material for an apparatus for use in hydrolysis of 2-hydroxy-4-methylthiobutanamide to obtain 2-hydroxy-4-methylthiobutanoic acid.

### Description of the Related Art

2-Hyroxy-4-methylthiobutanoic acid useful as an additive to a feed is known to be obtained by a method which comprises the steps of hydrating 2-hydroxy-4-methylthiobutanenitrile in the presence of sulfuric acid to obtain 2-hydroxy-4-methylthiobutanamide, and hydrolyzing 2-hydroxy-4-methylthiobutanamide to obtain 2-hydroxy-4-methylthiobutanoic acid (cf. JP-A-2007-238555).

The use of a conventional SUS 304, SUS 316 or the like as a material for an apparatus for use in this reaction has difficulties, in view of the conditions such as a concentration of sulfuric acid and temperature. Thus, a glass-lined or resin-lined apparatus is used.
However, the glass-lined or resin-lined apparatus has a complicated structure and thus is hard to manufacture, and also has problems such as its low impact resistance.

### SUMMARY OF THE INVENTION

An object of the present invention is to provide a highly impact-resistant and corrosion-resistant material which can be readily fabricated for an apparatus with a complicated structure, as a material for an apparatus for use in hydrolysis of 2-hydroxy-4-methylthiobutanamide for obtaining 2-hydroxy-4-methylthiobutanoic acid, in the process for producing 2-hydroxy-4-methylthiobutanoic acid from 2-hydroxy-4-methylthiobutanenitrile via 2-hydroxy-4-methylthiobutanamide.

The present invention provides a process for producing 2-hydroxy-4-methylthiobutanoic acid, comprising carrying out the step of hydrolyzing 2-hydroxy-4-methylthiobutanamide in the presence of sulfuric acid at a temperature of from 90 to 130°C with an apparatus composed of an alloy, as a material, wherein the alloy is an alloy which contains 16.0 to 22.0% by weight of a Cr element, 16.0 to 22.0% by weight of a Mo element, 1.0 to 2.5% by weight of a Ta element and a Ni element as the rest, an alloy which contains 26.0 to 32.0% by weight of a Mo element and a Ni element as the rest, or an alloy which contains 0.12 to 0.25% by weight of a Pd element and a Ti element as the rest.

According to the present invention, there is provided a highly impact-resistant and corrosion-resistant material which can be readily fabricated for an apparatus with a complicated structure, as a material for an apparatus for use in hydrolysis of 2-hydroxy-4-methylthiobutanamide for obtaining 2-hydroxy-4-methylthiobutanoic acid, in the process for producing 2-hydroxy-4-methylthiobutanoic acid from 2-hydroxy-4-methylthiobutanenitrile via 2-hydroxy-4-methylthiobutanamide. Thus, 2-hydroxy-4-methylthiobutanoic acid can be reliably produced without any corrosion or damage of the apparatus.

### Detailed Description of the Invention

2-Hydroxy-4-methylthiobutanoic acid (hereinafter optionally referred to as HMTBA) is typically produced by hydrating 2-hydroxy-4-methylthiobutanenitrile (hereinafter optionally referred to as HMTBN) in the presence of sulfuric acid usually at a temperature of from about 40 to about 70°C to obtain 2-hydroxy-4-methylthiobutanamide (hereinafter optionally referred to as HMTBAA), and adding water to 2-hydroxy-4-methylthiobutanamide to thereby hydrolyze the same usually at a temperature of from about 90 to about 130°C.
For example, HMTBN is industrially produced by reacting acrolein with methyl mercaptan to obtain 3-methylthiopropionaldehyde, and reacting 3-methylthiopropionaldehyde with hydrogen cyanide.

Generally, HMTBAA is produced by feeding HMTBN, water and sulfuric acid to a hydration tank to hydrate HMTBN.
Fed to the hydration tank are ususally about 20 to about 70 parts by weight, preferably about 25 to about 50 parts by weight of water, per 100 parts by weight of HMTBN, and about 0.5 to about 1 mol in total, preferably about 0.6 to about 0.8 mol in total, of sulfuric acid, per 1 mol of the HMTBN.

It is also possible to feed water as a pre-mixture thereof with HMTBN and/or sulfuric acid, in other words, as an aqueous HMTBN solution and/or an aqueous sulfuric acid solution, to the hydration tank. Preferably, water is fed as an aqueous sulfuric acid solution to the hydration tank.
The hydration reaction is carried out usually at a temperature of about 40 to about 70°C for about 1 to about 3 hours. After the reaction, the reaction solution is aged. In the reaction solution, HMTBA is produced as a result of hydrolysis of a part of HMTBAA.

Then, the reaction solution containing HMTBAA as a main component, obtained in the hydration tank, is fed to a hydrolysis tank, and water is added to the reaction solution to thereby hydrolyze HMTBAA to produce HMTBA.
About 100 to about 200 parts by weight of water is usually fed per 100 parts by weight of the aqueous sulfuric acid solution in the above-described reaction solution.
In the hydrolysis tank, HMTBAA is hydrolyzed with water and sulfuric acid to produce HMTBA and concurrently to by-produce ammonium bisulfate (NH₄HSO₄) and ammonium sulfate ((NH₄)₂SO₄). After the addition of water and heating, the hydrolysis reaction is carried out usually at a temperature of from about 90 to about 130°C for about 2 to about 6 hours.

The apparatus for use in hydrolysis refers to a hydrolysis tank, a pipe combined thereto so as to contact a hydration reaction mass, a heat exchanger or the like.
In the present invention, as a material for the apparatus for use in hydrolysis of HMTBAA to obtain HMTBA, there is used an alloy which contains 16.0 to 22.0% by weight of a Cr element, 16.0 to 22.0% by weight of a Mo element, 1.0 to 2.5% by weight of a Ta element and a Ni element as the rest, an alloy which contains 26.0 to 32.0% by weight of a Mo element and a Ni element as the rest, or an alloy which contains 0.12 to 0.25% by weight of a Pd element and a Ti element as the rest.
As a commercially available alloy which contains 16.0 to 22.0% by weight of a Cr element, 16.0 to 22.0% by weight of a Mo element, 1.0 to 2.5% by weight of a Ta element and a Ni element as the rest, there is exemplified MAT® 21 (Cr: 19% by weight, Mo: 19% by weight, Ta: 1.8% by weight, and Ni: the rest). As a commercially available alloy which contains 26.0 to 32.0% by weight of a Mo element, and a Ni element as the rest, there is exemplified Hastelloy® B-2 (Mo: 28% by weight, and Ni: the rest).
In this connection, as a material for an apparatus for use in hydration of HMTBN to obtain HMTBAA, there can be used MAT 21 or Hastelloy B-2 as described above.

The reaction solution containing HMTBA, obtained in the hydrolysis tank, is usually distilled to remove low boiling point components from the reaction solution.
The distillation is carried out usually at a temperature of from about 80 to about 120°C under a pressure of from about 50 to about 150 kPa to remove by-produced low boiling point components such as dimethyl sulfide, dimethyl disulfide and formic acid. The low boiling point components are removed as a distillate usually at a rate of about 1 to about 4% by weight relative to the reaction solution, as required.
The removal of the low boiling point components may be done after neutralization or phase separation as will be described later.

Then, an alkali is added to the reaction solution containing HMTBA, from which the low boiling point components have been removed, to thereby neutralize the reaction solution and separate the reaction solution into an organic phase containing HMTBA, and an aqueous phase containing water and an inorganic salt (containing ammonium bisulfate and ammonium sulfate). The neutralization and the phase separation are conducted, using a mixer-settler type liquid-liquid extractor in which a stirrer tank and a phase-separation tank are combined as one set.

As the alkali, for example, sodium hydroxide, sodium hydrogencarbonate, sodium carbonate or the like is used in the form of an aqueous solution. The alkali is fed usually at a rate of from about 0.5 to about 1.2 mol, preferably from about 0.6 to about 0.8 mol, per 1 mol of ammonium bisulfate in the above-described reaction solution. The addition rate of the alkali may be controlled by a hydrogen-ion concentration (pH) of the reaction solution admixed with the alkali.
The neutralization reaction is carried out usually at a temperature of from about 15 to about 120°C, preferably from about 30 to about 110°C, for about 0.1 to 3 hours, preferably about 0.1 to about 2 hours.

After the neutralization, the reaction solution is left to stand still in the phase-separation tank so as to phase-separate the reaction solution into an organic phase as an upper layer and an aqueous phase as a lower layer (liquid separation). The temperature for this separation is typically from about 30 to about 110°C.

The organic phase separated from the aqueous phase contains typically about 40 to about 60% by weight of HMTBA, about 20 to about 30% by weight of water and about 10 to about 30% by weight of an inorganic salt.
This organic phase is concentrated to remove the remaining water. For example, the concentration is done in a concentration tank usually at a temperature of from about 60 to about 150°C under a pressure of from about 1 to about 20 kPa, so that the water in the organic phase is reduced to usually about 5% by weight or less, preferably about 2% by weight or less, more preferably about 1% by weight or less. By this concentration, the sulfate-ion concentration and kinematic viscosity of a product as will be described later can be decreased.
The inorganic salt is precipitated from the organic phase by this concentration, and the resulting organic phase forms a slurry.

When the residence time of the organic phase in the concentration tank is set to about 0.5 hour or longer, HMTBA is converted into an oligomer (which is mainly a dimer containing small amounts of a trimer and a tetramer). By doing so, the solubility of the inorganic salt in the organic phase is lowered, so that the concentration of the inorganic salt in the organic phase can be lowered. Preferably, the temperature, pressure and residence time are selected so that the weight ratio of the monomer of HMTBA to the oligomer of HMTBA can be about 2 to about 4. Such a concentration is effective to increase the particle size of the inorganic salt precipitated in the organic phase, so that a solid-liquid separation efficiency is improved during a solid-liquid separation as will be described later. Thus, the removal of the inorganic salt is facilitated.

Then, the resultant slurry of the organic phase is cooled to usually about 60 to about 120°C, using a heat exchanger or the like. After that, the slurry is separated into a liquid component containing the organic phase and a solid component (or a residue) containing the precipitated inorganic salt.
Generally, the solid-liquid separation is carried out, using a solid-liquid separator such as a decanter type centrifugal separator.
The separated liquid component comprises HMTBA (containing the oligomer produced during the concentration) as a main component. If needed, water is added to the liquid component to provide a product of HMTBA which contains usually about 88 to about 90% by weight of HMTBA, about 10 to about 12.5% by weight of a moisture content and very small amounts of other components.

The separated solid component contains typically about 20 to about 60% by weight of HMTBA, and thus is usually admixed with water to dissolve the inorganic salt, so as to phase-separate the solid component into an aqueous phase containing the inorganic salt, and an organic phase. This organic phase is mixed into the former organic phase obtained by the above-described neutralization and phase-separation, for recovery.

### Examples

A hydrolysis reaction mass from 2-hydroxy-4-methylthiobutanamide to 2-hydroxy-4-methylthiobutanoic acid was subjected to a corrosion test for the following metallic material: the hydrolysis reaction mass (HMTBA: 41% by weight, water: 35% by weight, NH₄HSO₄: 13% by weight, and (NH₄)₂SO₄: 11% by weight; a mixture composition of an oil phase and an aqueous phase) and a test piece of the following metallic material (a flat plate of 25 mm in length X 20 mm in width X 2 mm in thickness) were put in a glass-made autoclave; the gas phase zone was purged with a nitrogen gas; and the autoclave was heated to 110°C and was maintained at the same temperature for 20 hours. After that, the test piece was removed, rinsed and dried. Then, the reduced weight of the test piece was measured to determine a corrosion rate. The results are shown in Table 1.

### (Test Piece)

Test piece 1: MAT® 21 (Cr: 18.4% by weight, Mo: 18.5% by weight, Ta: 1.9% by weight, and Ni: the rest)
Test piece 2: Hastelloy® B-2 (Mo: 27.7% by weight, and Ni: the rest)
Test piece 3: Ti-Pd (Pd: 0.17% by weight, and Ti: the rest)

**[Table 1]**

| No. | Test piece | Corrosion rate (mm/year) | Corroded state |
|---|---|---|---|
| 1 | Test piece 1 | 0.004 | Not corroded, and not damaged |
| 2 | Test piece 2 | 0.021 | Not corroded, and not damaged |
| 3 | Test piece 3 | 0.017 | Not corroded, and not damaged |

It was confirmed that Test piece 1, Test piece 2 and Ti-Pd did not corrode due to the hydrolysis reaction mass. An apparatus with a complicated structure can be readily constructed, using any of these alloys, and 2-hydroxy-4-methylthiobutanoic acid can be reliably produced without any corrosion or damage of the apparatus.

## Claims

1. A process for producing 2-hydroxy-4-methylthiobutanoic acid, comprising carrying out the step of hydrolyzing 2-hydroxy-4-methylthiobutanamide in the presence of sulfuric acid at a temperature of from 90 to 130°C with an apparatus composed of an alloy, as a material, which contains 16.0 to 22.0% by weight of a Cr element, 16.0 to 22.0% by weight of a Mo element, 1.0 to 2.5% by weight of a Ta element and a Ni element as the rest, an alloy which contains 26.0 to 32.0% by weight of a Mo element and a Ni element as the rest, or an alloy which contains 0.12 to 0.25% by weight of a Pd element and a Ti element as the rest.

2. A process according to Claim 1, comprising
the step of hydrating 2-hydroxy-4-methylthiobutanenitrile in the presence of sulfuric acid at a temperature of from 40 to 70°C to obtain 2-hydroxy-4-methylthiobutanamide; and the hydrolyzing step.

3. The process of Claim 1 or 2, wherein the alloy is an alloy which contains 16.0 to 22.0% by weight of a Cr element, 16.0 to 22.0% by weight of a Mo element, 1.0 to 2.5% by weight of a Ta element and a Ni element as the rest.

4. The process of Claim 1, wherein the alloy is an alloy which contains 26.0 to 32.0% by weight of a Mo element, and a Ni element as the rest.

5. Use of an alloy as a material for an apparatus used in the process of producing 2-hydroxy-4-methylthiobutanoic acid by hydrolyzing 2-hydroxy-4-methylthiobutanamide in the presence of sulfuric acid at a temperature of from 90 to 130°C.
